# EUROPEAN PATENT APPLICATION

(11) **EP 4 039 233 A1**
(43) Date of publication of application: **10.08.2022**
(21) Application number: 21183200.1
(22) Date of filing: 01.07.2021
(51) Int. Cl.: A61F 7/00, A61F 7/02, A61H 23/02

(54) **ELASTIC BAND EQUIPPED WITH VIBRATING CELLS AND PELTIER CELLS**

(30) Priority: 05.02.2021 IT 202100002405
(71) Applicant: H-Opera S.r.l., 84084 Fisciano (SA) (IT)
(72) Inventor: LONGO, Donato, 84091 Battipaglia (SA) (IT); MAINARDI, Luigi, 84091 Battipaglia (SA) (IT); CATALDO, Emilio, 84091 Battipaglia (SA) (IT); TORELLI, Giovanni, 84131 SALERNO (IT)
(74) Representative: Conversano, Gabriele

(57) **Abstract**

Elastic band (1) for bandages, comprising at least a Peltier cell and at least a means for application of vibrations positioned so that it is in contact with the skin when the elastic band is worn, and characterized in that it comprises further a sensor configured to measure the pressure exerted by the elastic band, and electronic control means configured to acquire the signal detected by said pressure monitor and to control said at least one Peltier cell and said at least one means for application of vibrations as a function of the signal detected by said pressure monitor.

## Description

The present invention relates to a device to be used for the treatment of muscle, tendinous or bone traumas, and in particular to a device configured to stimulate by heat and vibrations a body area subjected to a trauma.

### State of the art

At the state of the art there are known elastic bands comprising Peltier cells and mechanical stimulation means. Two examples ae described in documents US2013085552A1 and US2018369064A1. In US2013085552 it is described a device with Peltier cells controlled by a microprocessor and arranged on a thermally conductive surface resting on the area to be treated and configured to exchange thermal energy with the area to be treated and to stimulate the area to be treated mechanically by means of vibrations.

Anyway, the just cited examples and other ones known at the state of the art are limited since they do not allow neither to receive a feedback about the efficacy of the applied treatment nor to modulate automatically the intensity of the applied treatment as a function of the evolution of the pathology.

### Technical problem

So, a technical problem linked to the devices known at the state of the art is that the only way to check the evolution of the trauma is to remove the band. However, removing the band provides another undesired stress to the area subjected to trauma. Moreover, if the band was positioned by ax expert therapist, it would be difficult to repeat the operation in the same way unless he is there, and this needs that the patient has to go to the place where the therapist works. With the systems known at the state of the art it is substantially needed to go to the therapist to check if the trauma is passed, since the check will be visual and the new band will be positioned by the expert therapist.

### Aim of the invention

Aim of the present invention is an elastic band for bandages which overcomes the limits linked to the embodiments of the state of the art, and in particular which allows to obtain a feedback about the evolution of the trauma without needing to remove the band or to go to an expert therapist.

### Description of the invention

The device according to the invention is an elastic band (1) for bandages, comprising at least a Peltier cell and at least a means for application of vibrations positioned so that it is in contact with the skin when the elastic band is worn, and characterized in that it comprises further a sensor configured to measure the pressure exerted by the elastic band, and electronic control means configured to acquire the signal detected by said pressure monitor and to control said at least one Peltier cell and said at least one means for application of vibrations as a function of the signal detected by said pressure monitor.

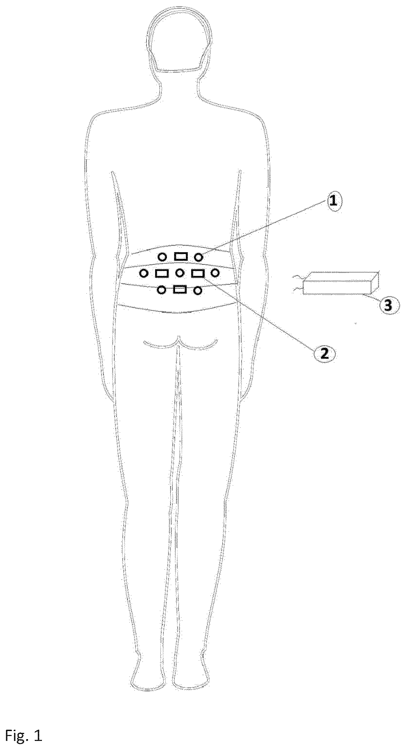

Moreover, the elastic band comprises preferably a plurality of Peltier cells and a plurality of means for application of vibration, arranged according to regular geometrical patterns and preferably in a plurality of rows, as it is shown in figure 1, which shows Peltier cells and means for application of vibration arranged alternatively to each other. Said elastic band is also provided with fastening means which allow its fastening in a plurality of predetermined positions, so that once the band is fixed, a difference in the pressure detected can depend only on the lower or higher swelling of the area under treatment.

In a first embodiment, said sensor configured to measure the pressure exerted by the elastic band comprises at least an extensometer configured to detect the elastic deformation of said band.

In a second embodiment said sensor comprises a flexible case filled with gas and a pressure sensor configured to detect the pressure of the gas inside said case, said case being interposed between the band and the area to be treated.

After describing the device, it is now possible to describe its functioning.

Firstly, it is to be specified that the use of a Peltier cell and of means for application of vibrations are functional to the application of heat and mechanical vibrations to the portion of the body subjected to the trauma.

The effect both of heat and vibrations is known per se at the state of the art.

Let's consider a person suffering from Achilles tendinitis: in addition to the pain, in most cases he will feel hypovascularization of foot and leg, also due to a possible brace which has to be applied to limit the movement of the limb. The application of an elastic band comprising vibrating cells and Peltier cells allows, as it is known, to favour the vascularization of the tissues interested by the trauma and the adjacent ones, thus limiting also the classic swelling, numbness, tingling which can limit the patient deambulation. The device can be activated once it is worn by the patient at the area subjected to the trauma, with the pressure monitors, the Peltier cells and the mass vibration motors arranged at the area interested by the trauma.

The device according to the invention is configured to carry out the method comprising the following steps.

100) acquiring a plurality of pressure measurements from said sensor in a predetermined time interval, in order to determine a starting pressure value.

In other words, said starting pressure value indicates the status of the trauma at the beginning of the treatment. Higher or lower swelling determine a higher or lower pressure on the elastic band in the area interested by the trauma.

So, in this step, it is carried out a calibration of the starting pressure level exerted by the band on the area where the pressure monitors, Peltier cells and eccentric mass vibration motors are provided.

Preferably, in this step, the patient carries out a series of movements of the limb interested by the trauma for a short time, for example 1 minute. During their execution, the control means acquire a series of pressure measurements, and calculate an average of the pressure value detected.

200) applying a plurality of cycles of treatment comprising heat transfer by means of Peltier cells and/or application of vibrations, with predetermined intensity, frequency and temperature. It is clear that, the therapist decides the duration, intensity and frequency as well as the temperature of the treatment, as a function of the area interested by the trauma and of the severity of the same.

Anyway, preferably, in order to maximize the efficacy of the treatment and to simulate the effect obtained by means of a manual massage, the device is configured to activate sequentially each one of said Peltier cells and said vibrating cells along a predetermined shifting direction. It is clear that the preferred shifting direction is a function of the anatomical area interested by the trauma.

300) periodically monitoring the pressure acquired by said sensor, and reducing the intensity of the treatment limited as a function of the reduction of said pressure.

In a first embodiment, the reduction both of vibration amplitude and applied temperature is directly proportional to the reduction of pressure. This provides implicitly also a feedback to the patient who can monitor the evolution of his own trauma as a function of the intensity of the applied vibrations.

For example, the pressure value considered to reduce the intensity of the treatment is the average pressure value within 12 hours, compared with the last available average pressure value. At the end of the first 12 hours, said average pressure value is compared with a starting pressure value indicating the status of the trauma at the beginning of the treatment.

In a second embodiment the reduction of the intensity of vibrations occurs at predetermined staggered percentages, at predetermined time intervals, but only provided that the pressure detected by said pressure sensor is reduced.

For example, if pressure at the end of a 12 hours interval is reduced compared to the pressure in the previous interval, the device reduces the intensity of the applied vibrations of 20%.

From an embodiment point of view, it is to be specified that to optimize the positioning of cells as a function of the specific anatomy of the specific patient and the specific segment to be treated it is possible to adopt a realization method of said band by carrying out a 3D scan of the anatomical tract of interest, and to generate a printed reticular structure with additive manufacturing technologies which is provided with receiving pockets for vibrating cells and thermal cells positioned in specific critical points individuated by the doctor or therapist. Instead of using a standard band, a receiving structure of the system is printed *ad hoc* for any anatomical area.

## Claims

1. Elastic band (1) for bandages, comprising at least a Peltier cell and at least a means for application of vibrations positioned so that it is in contact with the skin when the elastic band is worn, and **characterized in that** it comprises further a sensor configured to measure the pressure exerted by the elastic band, and electronic control means configured to acquire the signal detected by said pressure monitor and to control said at least one Peltier cell and said at least one means for application of vibrations as a function of the signal detected by said pressure monitor.

2. Elastic band according to claim 1, **characterized in that** it comprises further a plurality of Peltier cells and a plurality of means for application of vibration, arranged according to regular geometrical patterns and preferably in a plurality of rows.

3. Elastic band according to claim 1, comprising further fastening means which allow its fastening in a plurality of predetermined positions, so that once the band is fixed, a difference in the pressure detected can depend only on the lower or higher swelling of the area under treatment.

4. Elastic band according to any one of the preceding claims, **characterized in that** said sensor configured to measure the pressure comprises at least an extensometer configured to detect the elastic deformation of said band.

5. Elastic band according to any one of claims 1 to 3, **characterized in that** said sensor comprises a flexible case filled with gas and a pressure sensor configured to detect the pressure of the gas inside said case, said case being interposed between the band and the area to be treated.

6. Elastic band according to any one of the preceding claims, **characterized in that** it is configured to carry out the method comprising the following steps:
100) acquiring a plurality of pressure measurements from said sensor in a predetermined time interval, in order to determine a starting pressure value;
200) applying a plurality of cycles of treatment comprising heat transfer by means of Peltier cells and/or application of vibrations, with predetermined intensity, frequency and temperature;
300) periodically monitoring the pressure acquired by said sensor, and reducing the intensity of the treatment limited as a function of the reduction of said pressure.

7. Elastic band according to claim 6, **characterized in that** it is configured to activate sequentially each one of said Peltier cells and said vibrating cells along a predetermined shifting direction.

8. Elastic band according to claim 6 or 7, **characterized in that** the reduction both of vibration amplitude and applied temperature is directly proportional to the reduction of pressure.

9. Elastic band according to claim 6 or 7, **characterized in that** the reduction of intensity of the vibrations occurs at predetermined staggered percentages, at predetermined time intervals, but only provided that the pressure detected by said pressure sensor at point 300) is reduced with respect to the previously detected pressure.
